# EUROPEAN PATENT SPECIFICATION

(11) **EP 3 848 347 B1**
(45) Date of publication and mention of the grant of the patent: **20.09.2023**
(21) Application number: 21160064.8
(22) Date of filing: 11.06.2010
(51) Int. Cl.: C07C 51/41, C07C 51/377, C07C 55/14, C07C 253/00, C07C 255/04, C07C 209/00, C07C 211/12, C07D 309/30, C07D 201/08, C07D 223/10, C07C 51/235, C08G 63/16, C08G 69/26, C08G 69/14, C08G 69/36

(54) **PRODUCTION OF GLUCARIC ACID AND DERIVATIVES FROM GLUCOSE**
HERSTELLUNG VON GLUCARSÄURE UND DERIVATEN AUS GLUKOSE
PRODUCTION D'ACIDE GLUCARIQUE ET DE DÉRIVÉS À PARTIR DE GLUCOSE

(30) Priority: 13.06.2009 US 26841409 P; 05.03.2010 US 31119010 P
(43) Date of publication of application: 14.07.2021
(62) Divisional of application: 18183021.7
(73) Proprietor: Archer-Daniels-Midland Company, Decatur, IL 62526 (US)
(72) Inventor: BOUSSIE, Thomas R., Decatur, IL 62525 (US); DIAS, Eric L., Decatur, IL 62526 (US); FRESCO, Zachary M., Decatur, IL 62526 (US); MURPHY, Vincent J., Decatur, IL 62526 (US); SHOEMAKER, James, Decatur, IL 62525 (US); ARCHER, Raymond, Decatur, IL 62526 (US); JIANG, Hong, Decatur, IL 62525 (US)
(74) Representative: Potter Clarkson

(56) References cited:
- BESSON M ET AL: "Oxidation of glucose and gluconate on Pt, Pt Bi, and Pt Au catalysts", RECUEIL DES TRAVAUX CHIMIQUES DES PAYS, ELSEVIER SCIENCE PUBLISHERS, AMSTERDAM, NL, vol. 115, no. 4, 1 January 1996 (1996-01-01), pages 217-221, XP008131910, ISSN: 0165-0513, DOI: 10.1002/RECL.19961150405
- ABBADI A ET AL: "Effect of pH in the Pt-catalyzed oxidation of D-glucose to D-gluconic acid", JOURNAL OF MOLECULAR CATALYSIS A: CHEMICAL, ELSEVIER, AMSTERDAM, NL, vol. 97, no. 2, 3 April 1995 (1995-04-03), pages 111-118, XP002617660, ISSN: 1381-1169, DOI: 10.1016/1381-1169(94)00078-6 [retrieved on 2000-04-21]

## Description

### FIELD OF THE INVENTION

The present invention generally relates to processes for the chemocatalytic conversion of a glucose source to glucaric acid or lactone thereof.

### BACKGROUND OF THE INVENTION

Crude oil is currently the source of most commodity and specialty organic chemicals. Many of these chemicals are employed in the manufacture of polymers and other materials. Examples include ethylene, propylene, styrene, bisphenol A, terephthalic acid, adipic acid, caprolactam, hexamethylene diamine, adiponitrile, caprolactone, acrylic acid, acrylonitrile, 1,6-hexanediol, 1,3-propanediol, and others. Crude oil is first refined into hydrocarbon intermediates such as ethylene, propylene, benzene, and cyclohexane. These hydrocarbon intermediates are then typically selectively oxidized using various processes to produce the desired chemical. For example, crude oil is refined into cyclohexane which is then selectively oxidized to "KA oil" which is then further oxidized for the production of adipic acid, an important industrial monomer used for the production of nylon 6,6. Many known processes are employed industrially to produce these petrochemicals from precursors found in crude oil. For example, see Ullmann's Encyclopedia of Industrial Chemistry, Wiley 2009 (7th edition).

For many years there has been an interest in using biorenewable materials as a feedstock to replace or supplement crude oil. See, for example, Klass, Biomass for Renewable Energy, Fuels, and Chemicals, Academic Press, 1998. Moreover, there have been efforts to produce adipic acid from renewable resources using processes involving a combination of biocatalytic and chemocatalytic processes. See, for example, "Benzene-Free Synthesis of Adipic Acid", Frost et al. Biotechnol. Prog. 2002, Vol. 18, pp. 201-211, and U.S. Patent Nos. 4,400,468, and 5,487,987.

One of the major challenges for converting biorenewable resources such as carbohydrates (e.g. glucose derived from starch, cellulose or sucrose) to current commodity and specialty chemicals is the selective removal of oxygen atoms from the carbohydrate. Approaches are known for converting carbon-oxygen single bonds to carbon-hydrogen bonds. See, for example: U.S. Patent No. 5,516,960; U.S. Patent App. Pub. US2007/0215484 and Japanese Patent No. 78,144,506. Each of these known approaches suffers from various limitations and we believe that, currently, none of such methods are used industrially for the manufacture of specialty or industrial chemicals.

Thus, there remains a need for new, industrially scalable methods for the selective and commercially-meaningful conversion of carbon-oxygen single bonds to carbon-hydrogen bonds, especially as applied in connection with the production of chemicals from polyhydroxyl-containing substrates (e.g., glucaric acid), and especially for the production of chemicals from polyhydroxyl-containing biorenewable materials (e.g., glucose derived from starch, cellulose or sucrose) to important chemical intermediates such as adipic acid. BESSON, M.; FLECHE, G.; FUERTES, P.; GALLEZOT, P.; LAHMER, F., "Oxidation of glucose and gluconate on Pt, Pt Bi, and Pt Au catalysts", RECUEIL DES TRAVAUX CHIMIQUES DES PAYS-BAS, (1996), vol. 115, no. 4 summarizes that glucose and gluconate aqueous solutions were oxidized with air on active charcoal-supported, platinum catalysts; some catalysts were promoted with bismuth or gold. ABBADI, A.; VAN BEKKUM, H., "Effect of pH in the Pt-catalyzed oxidation of D-glucose to D-gluconic acid", JOURNAL OF MOLECULAR CATALYSIS A: CHEMICAL, (1995), vol. 97 summarizes the pH effect on the Pt-catalyzed selective heterogeneous oxidation of D-glucose to D-gluconic acid.

### SUMMARY OF THE INVENTION

The present invention is directed to a process for preparing glucaric acid or lactone thereof, the process comprising: reacting glucose with oxygen in the presence of heterogeneous oxidation catalyst comprising Pt to convert at least a portion of the glucose to glucaric acid or lactone thereof, wherein the pH of the reaction mixture is less than 7, wherein the reaction is conducted in the substantial absence of added base, wherein the reaction mixture is maintained at a temperature from 70°C to 150°C and under a partial pressure of oxygen that is at least 60 psia (414 kPa), and wherein the heterogeneous oxidation catalyst comprises a catalyst support selected from the group consisting of carbon, alumina, silica, titania, zirconia, and zeolite.
Also described herein are processes for preparing an adipic acid product from polyhydroxyl-containing biorenewable materials..

As described herein, the process for preparing an adipic acid product comprises reacting, in the presence of a hydrodeoxygenation catalyst and a halogen source, a hydrodeoxygenation substrate and hydrogen to convert at least a portion of the hydrodeoxygenation substrate to an adipic acid product, wherein the hydrodeoxygenation substrate comprises a compound of formula I 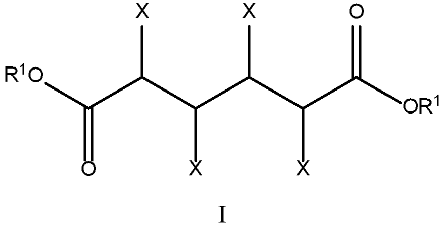 wherein X is independently hydroxyl, oxo, halo, acyloxy or hydrogen provided that at least one X is not hydrogen and R¹ is independently a salt-forming ion, hydrogen, hydrocarbyl, or substituted hydrocarbyl; or a mono- or di-lactone thereof.

As described herein, the process for preparing an adipic acid product comprises converting at least a portion of a glucose source to a hydrodeoxygenation substrate comprising glucaric acid or derivative thereof, and converting at least a portion of the glucaric acid or derivative to an adipic acid product.

The process of the invention comprises reacting glucose with a source of oxygen in the presence of an oxidation catalyst and in the substantial absence of added base as defined in Claim 1.

Also described herein are processes for preparing glucaric acid by reacting glucose with oxygen in the presence of an oxidation catalyst, wherein at least a portion of the glucose is solubilized with a weak carboxylic acid, preferably acetic acid.

Also described herein are processes for the preparation of industrial chemicals such as adiponitrile, hexamethylene diamine, caprolactam, caprolactone, 1,6-hexanediol, adipate esters, polyamides (e.g., nylons) and polyesters from an adipic acid product obtained from processes for the chemocatalytic conversion of a glucose source, which may include, for example, the catalytic hydrodeoxygenation of glucaric acid or derivatives thereof.

Also described herein are adipic acid products, polyamides, polyesters and caprolactam produced at least in part from adipic acid product produced by the hydrodeoxygenation of a hydrodeoxygenation substrate, and, more particularly, from glucaric acid or derivative thereof.

Other objects and features will become apparent and/or will be pointed out hereinafter.

### DESCRIPTION OF THE PREFERRED EMBODIMENTS

The present invention relates to processes for the catalytic production of glucaric acid from glucose. The process comprising: reacting glucose with oxygen in the presence of heterogeneous oxidation catalyst comprising Pt to convert at least a portion of the glucose, such as at least 50% of the glucose, to glucaric acid or lactone thereof, wherein the pH of the reaction mixture is less than 7, wherein the reaction is conducted in the substantial absence of added base, wherein the reaction mixture is maintained at a temperature from 70°C to 150°C and under a partial pressure of oxygen that is at least 60 psia (414 kPa), and wherein the heterogeneous oxidation catalyst comprises a catalyst support selected from the group consisting of carbon, alumina, silica, titania, zirconia, and zeolite.
. Conducting the oxidation reaction in the substantial absence of added base facilitates product recovery and improves process economics. Further, this reaction can be conducted in the presence of a weak carboxylic acid, such as acetic acid, in which at least a portion of the glucose is solubilized. Moreover, preferred oxidation catalysts and/or oxidation reaction conditions provide yields of glucaric acid in excess of 60%, and up to 65% or more.

As described herein, an adipic acid product prepared in accordance with the processes may be converted, according to processes known in the art, to various other industrially significant chemicals including, for example, adiponitrile, caprolactam, caprolactone, hexamethylene diamine, 1,6-hexanediol, adipate esters, polyamides (e.g., nylon) or polyesters. Thus, adiponitrile, caprolactam, caprolactone, hexamethylene diamine, 1,6-hexanediol, adipate esters, polyamides (e.g., nylon) and polyesters may be prepared from glucose derived from biorenewable sources.

### I. Feedstocks

Glucose can be obtained from various carbohydrate-containing sources including conventional biorenewable sources such as corn grain (maize), wheat, potato, cassava and rice as well as alternative sources such as energy crops, plant biomass, agricultural wastes, forestry residues, sugar processing residues and plant-derived household wastes. More generally, biorenewable sources that may be used in accordance with the present invention include any renewable organic matter that includes a source of carbohydrates such as, for example, switch grass, miscanthus, trees (hardwood and softwood), vegetation, and crop residues (e.g., bagasse and corn stover). Other sources can include, for example, waste materials (e.g., spent paper, green waste, municipal waste, etc.). Carbohydrates such as glucose may be isolated from biorenewable materials using methods that are known in the art. See, for example, Centi and van Santen, Catalysis for Renewables, Wiley-VCH, Weinheim 2007; Kamm, Gruber and Kamm, Biorefineries-Industrial Processes and Products, Wiley-VCH, Weinheim 2006; Shang-Tian Yang, Bioprocessing for Value-Added Products from Renewable Resources New Technologies and Applications, Elsevier B.V. 2007; Furia, Starch in the Food Industry, Chapter 8, CRC Handbook of Food Additives 2nd Edition CRC Press, 1973. See also chapters devoted to Starch, Sugar and Syrups within Kirk-Othmer Encyclopedia of Chemical Technology 5th Edition, John Wiley and Sons 2001. Also, processes to convert starch to glucose are known in the art, see, for example, Schenck, "Glucose and Glucose containing Syrups" in Ullmann's Encyclopedia of Industrial Chemistry, Wiley-VCH 2009. Furthermore, methods to convert cellulose to glucose are known in the art, see, for example, Centi and van Santen, Catalysis for Renewables, Wiley-VCH, Weinheim 2007; Kamm, Gruber and Kamm, Biorefineries-Industrial Processes and Products, Wiley-VCH, Weinheim 2006; Shang-Tian Yang, Bioprocessing for Value-Added Products from Renewable Resources New Technologies and Applications, Elsevier B.V. 2007.

### II. Preparation of Glucaric Acid

In accordance with the present invention, glucose is converted to, for example, glucaric acid.

Applicants have discovered that glucose may be converted to glucaric acid in high yield by reacting glucose with oxygen (as used herein, oxygen can be supplied to the reaction as air, oxygen-enriched air, oxygen alone, or oxygen with other constituents substantially inert to the reaction) in the presence of a heterogeneous oxidation catalyst comprising Pt and in the absence of added base according to the following reaction:

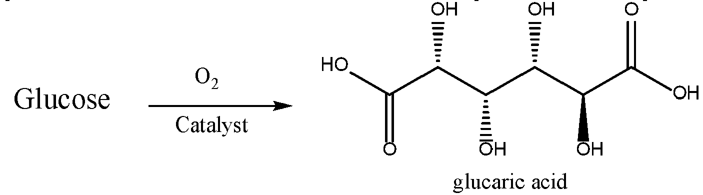

Surprisingly, conducting the oxidation reaction in the absence of added base and in accordance with the reaction conditions set forth herein, does not lead to significant catalyst poisoning effects and catalyst oxidation selectivity is maintained. In fact, catalytic selectivity can be maintained to attain glucaric acid yield in excess of 50%, even 60% and, in some instances, attain yields in excess of 65% or higher. The absence of added base advantageously facilitates separation and isolation of the glucaric acid, thereby providing a process that is more amenable to industrial application, and improves overall process economics by eliminating a reaction constituent. The "absence of added base" as used herein means that base, if present (for example, as a constituent of a feedstock), is present in a concentration which has essentially no effect on the efficacy of the reaction; i.e., the oxidation reaction is being conducted essentially free of added base. It has also been discovered that this oxidation reaction can also be conducted in the presence of a weak carboxylic acid, such as acetic acid, in which glucose is soluble. The term "weak carboxylic acid" as used herein means any unsubstituted or substituted carboxylic acid having a pKa of at least about 3.5, more preferably at least about 4.5 and, more particularly, is selected from among unsubstituted acids such as acetic acid, propionic acid or butyric acid, or mixtures thereof.

It has been further discovered that conducting the oxidation reaction under increased oxygen partial pressures and/or higher oxidation reaction mixture temperatures tends to increase the yield of glucaric acid when the reaction is conducted in the substantial absence of added base.

In these and various other embodiments, the initial pH of the reaction mixture is no greater than about 7, and typically is less than 7 such as, for example, 6 or less when a weak carboxylic acid is used to solubilize at least a portion of the glucose. In accordance with the present invention, the initial pH of the reaction mixture is the pH of the reaction mixture prior to contact with oxygen in the presence of an oxidation catalyst. It is expected that the pH of the reaction mixture after oxygen contact will vary as the reaction proceeds. It is believed that as the concentration of the glucaric acid increases (as the reaction proceeds) the pH will decrease from the initial pH.

Another advantage of the present invention is the essential absence of nitrogen as an active reaction constituent. Typically, nitrogen is employed in known processes as an oxidant such as in the form of nitrate, in many instances as nitric acid. The use of nitrogen in a form in which it is an active reaction constituent, such as nitrate or nitric acid, results in the need for NOₓ abatement technology and acid regeneration technology, both of which add significant cost to the production of glucaric acid from these known processes, as well as providing a corrosive environment which may deleteriously affect the equipment used to carry out the process. By contrast, for example, in the event air or oxygen-enriched air is used in the oxidation reaction of the present invention as the source of oxygen, the nitrogen is essentially an inactive or inert constituent. Thus, for example, in accordance with the present invention, an oxidation reaction employing air or oxygen-enriched air is a reaction conducted essentially free of nitrogen in a form in which it would be an active reaction constituent.

The temperature of the oxidation reaction mixture is from 70°C to 150°C, from 70°C to 140°C, or from 80°C to 120°C.

The partial pressure of oxygen is at least 60 psia (414 kPa). In various embodiments, the partial pressure of oxygen is up to about 1000 psia (6895 kPa), or more typically in the range of from about 15 psia (104 kPa) to about 500 psia (3447 kPa).

The oxidation reaction may be conducted in the presence of a solvent to glucose. Solvents suitable for the oxidation reaction include water and weak carboxylic acids such as acetic acid. Utilization of weak carboxylic acid as a solvent adds cost to the process which cost, as a practical matter, must be balanced against any benefits derived from the use thereof. Thus, suitable solvents for the present invention include water, mixtures of water and weak carboxylic acid, or weak carboxylic acid.

The oxidation reaction can be conducted in a batch, semi-batch, or continuous reactor design using fixed bed reactors, trickle bed reactors, slurry phase reactors, moving bed reactors, or any other design that allows for heterogeneous catalytic reactions. Examples of reactors can be seen in Chemical Process Equipment - Selection and Design, Couper et al., Elsevier 1990. It should be understood that glucose, oxygen, any solvent, and the oxidation catalyst may be introduced into a suitable reactor separately or in various combinations.

Catalysts suitable for the oxidation reaction ("oxidation catalyst") are heterogeneous catalysts, including solid-phase catalysts comprising one or more supported metals. The metal comprises platinum. Additional other metals may be present, including one or more d-block metals, alone or in combination with one or more rare earth metals (e.g. lanthanides), alone or in combination with one or more main group metals (e.g. Al, Ga, Tl, In, Sn, Pb or Bi). In general, the metals may be present in various forms (e.g., elemental, metal oxide, metal hydroxides, metal ions, etc.). Typically, the metal(s) at a surface of a support may constitute from about 0.25% to about 10%, or from about 1% to about 8%, or from about 2.5% to about 7.5% (e.g., 5%) of the total weight of the catalyst.

In various embodiments, the oxidation catalyst comprises a first metal (M1) and a second metal (M2) at a surface of a support, wherein the M1 metal is selected from the group consisting of palladium and platinum and the M2 metal is selected from the group consisting of d-block metals, rare earth metals, and main group metals, wherein the M1 metal is not the same metal as the M2 metal. In various preferred embodiments, the M1 metal is platinum and the M2 metal is selected from the group consisting of manganese, iron, and cobalt.

The M1:M2 molar ratio may vary, for example, from about 500:1 to about 1:1, from about 250:1 to about 1:1, from about 100:1 to about 1:1, from about 50:1 to about 1:1, from about 20:1 to about 1:1, or from about 10:1 to about 1:1. In various other embodiments, the M1:M2 molar ratio may vary, for example, from about 1: 100 to about 1:1, from about 1:50 to about 1:1, from about 1:10 to about 1:1, from about 1:5 to about 1:1, or from about 1:2 to about 1:1.

Moreover, the weight percents of M1 and M2 relative to the catalyst weight may vary. Typically, the weight percent of M1 may range from about 0.5% to about 10%, more preferably from about 1% to about 8%, and still more preferably from about 2.5% to about 7.5% (e.g., about 5%). The weight percent of M2 may range from about 0.25% to about 10%, from about 0.5% to about 8%, or from about 0.5% to about 5%.

In various other embodiments, a third metal (M3) may be added to produce a M1/M2/M3 catalyst wherein the M3 metal is not the same metal as the M1 metal and the M2 metal. In yet other embodiments a fourth metal (M4) may be added to produce a M1/M2/M3/M4 catalyst wherein the M4 metal is not the same metal as the M1 metal, the M2 metal or the M3 metal. The M3 metal and M4 metal may each be selected from the group consisting of d-block metals, rare earth metals (e.g. lanthanides), or main group metals (e.g. Al, Ga, Tl, In, Sn, Pb or Bi).

Suitable catalyst supports are selected from carbon, alumina, silica, titania, zirconia, and zeolite, and modifications, mixtures or combinations thereof. As described herein preferred support materials may be modified using methods known in the art such as heat treatment, acid treatment or by the introduction of a dopant (for example, metal-doped titanias, metal-doped zirconias (e.g., tungstated-zirconia), metal-doped cerias, and metal-modified niobias). Particularly preferred supports are carbon (which may be activated carbon, carbon black, coke or charcoal), alumina, zirconia, titania, zeolite and silica. In various embodiments, the support of the oxidation catalyst is selected from the group consisting of carbon, zirconia, zeolite, and silica.

The metals may be deposited using procedures known in the art including, but not limited to incipient wetness, ion-exchange, deposition-precipitation, and vacuum impregnation. When two or more metals are deposited on the same support, they may be deposited sequentially or simultaneously. In various embodiments, following metal deposition, the catalyst is dried at a temperature of at least about 50°C, more typically at least about 120°C for a period of time of at least about 1 hour, more typically 3 hours or more. In these and other embodiments, the catalyst is dried under sub-atmospheric pressure conditions. In various embodiments, the catalyst is reduced after drying (e.g., by flowing 5% H₂ in N₂ at 350 °C for 3 hours). Still further, in these and other embodiments, the catalyst is calcined, for example, at a temperature of at least about 500°C for a period of time (e.g., at least about 3 hours).

The reaction product of the oxidation step will, as described above, yield glucaric acid in considerable and heretofore unexpected fraction, but may also yield derivatives thereof, such as glucarolactones. These glucarolactones, like glucaric acid, constitute hydrodeoxygenation substrate which is particularly amenable to the production of adipic acid product as hereinafter described. Glucarolactones which may be present in the reaction mixture resulting from the oxidation step include mono and di-lactones such as D-glucaro-1,4-lactone, D-glucaro-6,3- lactone, and D-glucaro-1,4:6,3-dilactone. One advantage of higher concentrations of glucarolactones is further improvement in the economics of the hydrodeoxygenation step resulting from a reduction in the amount of water produced.

Glucaric acid produced in accordance with the above may be converted to various other glucaric acid derivatives, such as salts, esters, ketones, and lactones. Methods to convert carboxylic acids to such derivatives are known in the art, see, for example, Wade, Organic Chemistry 3rd ed, Prentice Hall 1995.

### EXAMPLES

The following non-limiting examples are provided to further illustrate the present invention.

Reactions were conducted in 1 mL glass vials housed in a pressurized vessel in accordance with the procedures described in the examples below. Product yields were determined using a Dionex ICS-3000 Chromatography system. For Example 1, the products were first separated on an lonpac^{©} AS11-HC column and then quantified by conductivity detection through comparison with calibration standards. For Example 2, the products were first separated on an Acclaim^{©} Organic Acid column and then quantified by a UV detector through comparison with calibration standards.

### EXAMPLE 1: GLUCOSE TO GLUCARIC ACID

Several catalysts were obtained from commercial vendors: Johnson Matthey 5% Pt/C (three examples; JM-23 [B103032-5, Lot #C-9090]; JM-25 [B103014-5, Lot #C9230]; and JM-27 [B-501032-5, Lot #C-9188]), Johnson Matthey 5% Pt/Al₂O₃ (two examples; JM-32 [B301013-5, Lot #C8959] and JM-33 [B301099-5, Lot #C9218]), and BASF Escat 2351 5% Pt/SiO₂ [Lot #A4048107]; and 1.5% Au/TiO₂ [Süd Chemie 02-10]. Other catalysts were prepared in accordance with the procedure described herein.

### Preparation of Supported Platinum Catalysts

Multiple portions of suitably concentrated aqueous Pt(NO₃)₂ solutions (Heraeus) were added to the appropriate support (wherein the total combined volume of the Pt(NO₃)₂ solutions was matched to equal to the pore volume of the chosen support) with agitation between additions. Post impregnation, the product was dried in a furnace at 120 °C for 12 hours, Material for catalyst testing was prepared by reduction under flowing 5 vol.% H₂ in N₂ for 3 hours at either 200 °C or 350 °C. Note that this procedure was used for all supports except carbon. See the later description for the preparation of a Pt/Carbon catalyst.

### Preparation of Pt/M2/Support Catalysts (M2 = Mn, Co, Fe, Re, Cu)

Approximately 7 - 8 mg of dried supported platinum catalyst (taken post drying but prior to reduction) was dispensed into an 8 x 12 array containing 1 mL glass vials. To select vials within the array, 6 - 7 µl (where the total addition volume was matched to equal to the pore volume of the support weighed into the vial) of suitably concentrated M2 stock solutions were added (M2 = Mn, Fe, Co, Re, Cu obtained from Strem or Sigma-Aldrich, see Table 1). Post M2 addition, the mixtures were agitated via a multi-tube vortexer to impregnate the supports. Post impregnation, the glass vial arrays of Pt/M2/Support catalysts were dried in a furnace at 120 °C for 1 hour, followed by calcination at 500 °C for 3 hours followed by reduction under flowing 5 vol. % H₂ in N₂ at either 200 °C or 350 °C for 3 hours. Note that this procedure was used to prepare all Pt/M2/Support catalysts with the exception of the 1.5 % Pt/1.5% Au/Titania catalyst. In this case Pt(NO₃)₂ solution was added to a dried sample of the commercial 1.5% Au/Titania catalyst [Süd Chemie 02-10] (wherein the total volume of the Pt(NO₃)₂ volume was matched to equal to the pore volume of the catalyst) with agitation, whereupon the material was dried in a furnace at 120 °C for 1 hour, followed by reduction under flowing 5 vol. % H₂ in N₂ at 350 °C for 3 hours.

### Preparation of 4 wt.% Pt/Carbon Catalyst

Multiple portions of suitably concentrated aqueous Pt(NO₃)₂ solution (Heraeus) were added to 2 g of dried Degussa HP-160 furnace black carbon (3.94 mL total addition volume) with agitation between additions. Post impregnation, the 4 wt.% Pt/Carbon was dried under vacuum for one hour at 50°C, followed by reduction under flowing 5 vol.% H₂ in N₂ for three hours at 350°C.

### Glucose to Glucaric Acid Reactions

Catalysts were dispensed into 1mL vials within a 96-well reactor insert (Symyx Solutions). The reaction substrate was D-glucose (Sigma-Aldrich, 0.552M in water). To each vial was added 250 µL of glucose solution. The vials were each covered with a Teflon pin-hole sheet, a silicone pin-hole mat and steel gas diffusion plate (Symyx Solutions). The reactor insert was placed in a pressure vessel and charged three times with oxygen to 100 psig with venting after each pressurization step. The reactor was then charged to 75 psig with oxygen, or to 500 psig with air, closed and placed on a shaker, heated at the designated temperature for the specified reaction time. After the reaction time had elapsed shaking was stopped and the reactor cooled to room temperature whereupon the reactors were vented. Samples for ion-chromatography (IC) analysis were prepared by adding to each reaction vial 750 µL of a 1.067 wt.% citric acid solution (as internal standard) then the plate was covered and mixed followed by centrifugation to separate catalyst particles. Each reaction sample was further diluted by performing two 20-fold dilutions then analyzed by Ion Chromatography. In some instances, HCl was used as alternative internal standard through the addition of 100 µL of 50 ppm solution during the second 20-fold dilution. The results are presented in Table 1.

**Table 1.**

| | **Catalyst (wt.% M2 wt.% Pt/Support)** | **M1 Precursor** | **M2 Precursor** | **Temp. (°C)** | **Time (Hours)** | **Catalyst Amount (mg)** | **Glucaric Acid Yield (%)** |
|---|---|---|---|---|---|---|---|
| 1 | 0.06% Mn 4% Pt / Silica Davisil 635 | Pt(NO₃)₂ | Mn(NO₃)₂ | 80 | 5 | 7 | 38 |
| 2 | 0.06% Fe 4% Pt / Silica Davisil 635 | Pt(NO₃)₂ | Fe(NO₃)₃ | 80 | 5 | 8 | 28 |
| 3 | 0.06% Co 4% Pt / Silica Davisil 635 | Pt(NO₃)₂ | Co(NO₃)₂ | 80 | 5 | 8 | 34 |
| 4 | 4% Pt / Silica Davisil 635 | Pt(NO₃)₂ | None | 80 | 5 | 8 | 34 |
| 5 | 4% Pt / Silica 5 µm Cariact | Pt(NO₃)₂ | None | 90 | 5 | 8 | 50 |
| 6 | 4% Pt / Silica 5 µm Cariact | Pt(NO₃)₂ | None | 90 | 8 | 8 | 66 |
| 7 | 4% Pt / Silica Merck 10180 | Pt(NO₃)₂ | None | 90 | 5 | 8 | 40 |
| 8 | 1.91%Re 4% Pt / Silica Merck 10180 | Pt(NO₃)₂ | HReO₄ | 90 | 5 | 8 | 39 |
| 9 | 0.65% Cu 4% Pt / Silica Merck 10180 | Pt(NO₃)₂ | Cu(NO₃)₂ | 90 | 5 | 8 | 39 |
| | | | (NH₄)₆Mo₇ | | | | |
| 10 | 0.10% Mo 4% Pt / Silica Merck 10180 | Pt(NO₃)₂ | O₂₄ | 90 | 5 | 8 | 38 |
| 11 | 4% Pt / Carbon Degussa HP-160 | Pt(NO₃)₂ | None | 80 | 5 | 8 | 53 |
| 12 | 4% Pt / Carbon Degussa HP-160 | Pt(NO₃)₂ | None | 90 | 8 | 8 | 60 |
| 13 | 5% Pt / C [JM-23] | | None | 80 | 5 | 10 | 52 |
| 14 | 5% Pt / C [JM-25] | | None | 80 | 5 | 10 | 57 |
| 15 | 5% Pt / C [JM-27] | | None | 80 | 5 | 10 | 57 |
| 16 | 5% Pt / Al₂O₃ [JM-32] | | None | 80 | 5 | 10 | 23 |
| 17 | 5% Pt/ Al₂O₃ [JM-33] | | None | 80 | 5 | 10 | 31 |
| 18 | 5% Pt / SiO₂ [BASF Escat 2351] | Pt(NO₃)₂ | None | 80 | 5 | 10 | 15 |
| 19 | 8% Pt / Zirconia Daiichi Kigenso Z-1044 | Pt(NO₃)₂ | None | 90 | 5 | 8 | 52 |
| 20 | 8% Pt / Zirconia Daiichi Kigenso Z-1628 | Pt(NO₃)₂ | None | 90 | 5 | 8 | 59 |
| 21 | 8% Pt / Zirconia Ceria Daiichi Kigenso Z-1006 | Pt(NO₃)₂ | None | 90 | 5 | 8 | 54 |
| 22 | 8% Pt / Ceria Daiichi Kigenso Z-1627 | Pt(NO₃)₂ | None | 90 | 5 | 8 | 17 |
| ^{b}23 | ^{a}4% Pt / Zeolite Zeolyst CP 811C-300 | Pt(NO₃)₂ | None | 100 | 5 | 8 | 39 |
| ^{b}24 | ^{a}4% Pt / Titania NorPro ST 61120 | Pt(NO₃)₂ | None | 100 | 5 | 8 | 30 |
| ^{b}24 | 1.5% Pt 1.5% Au / Titania [Süd Chemie 02-10] | | Pt(NO₃)₂ | 100 | 5 | 8 | 55 |
| ^{b}25 | 4% Pt 4% Au / Titania NorPro ST 61120 | AuCl₃ | Pt(NO₃)₂ | 100 | 5 | 8 | 32 |

| | | | | | | | |
|---|---|---|---|---|---|---|---|
| ^{a}These catalysts were calcined at 500 °C for 3 hours prior to reduction. ^{b}These reactions were run under 500 psig of air, all other reactions in Table 1 were run under 75 psig of O₂. Catalysts in examples 4-7, 11-12 were reduced at 200 °C under flowing 5 vol.% H₂ in N₂ for 3 hours. Catalysts in examples 1-3, 8-10, 19-25 were reduced at 350 °C under flowing 5 vol.% H₂ in N₂ for 3 hours. Commercial catalysts in examples 13-18 were screened directly. | | | | | | | |

## Claims

1. A process for preparing glucaric acid or lactone thereof, the process comprising:
reacting glucose with oxygen in the presence of heterogeneous oxidation catalyst comprising Pt to convert at least a portion of the glucose to glucaric acid or lactone thereof,
wherein the pH of the reaction mixture is less than 7,
wherein the reaction is conducted in the substantial absence of added base,
wherein the reaction mixture is maintained at a temperature from 70°C to 150°C and under a partial pressure of oxygen that is at least 60 psia (414 kPa), and
wherein the heterogeneous oxidation catalyst comprises a catalyst support selected from the group consisting of carbon, alumina, silica, titania, zirconia, and zeolite.

2. The process as set forth in claim 1, wherein at least a portion of the glucose is converted to a glucarolactone.

3. The process as set forth in claim 1 or 2, wherein the catalyst support comprises titania.

4. The process as set forth in any one of claims 1 to 3, wherein the catalyst support comprises zirconia.

5. The process as set forth in any one of claims 1 to 4, wherein the catalyst support comprises carbon.

6. The process as set forth in any one of claims 1 to 5, wherein the catalyst support comprises carbon black.

7. The process as set forth in any one of claims 1 to 6, wherein Pt constitutes from 0.25% to 10% of the total weight of the heterogeneous oxidation catalyst.

8. The process as set forth in any one of claims 1 to 7, wherein the reaction mixture is maintained at a temperature from 80°C to 120°C.

9. The process as set forth in any one of claims 1 to 8, wherein the partial pressure of oxygen is up to 1000 psia (6895 kPa).

10. The process as set forth in any one of claims 1 to 9, wherein the glucose is obtained from a carbohydrate source.

11. The process as set forth in any one of claims 1 to 10, wherein the reaction mixture is essentially free of nitrogen as an active reaction constituent.

12. The process as set forth in any one of claims 1 to 11, wherein the initial pH of the reaction mixture is less than 6.

13. The process as set forth in any one of claims 1 to 12, wherein the reaction is conducted in one or more continuous fixed bed reactors.

14. The process as set forth in any one of claims 1 to 13, wherein the catalyst comprises a first metal (M1) and further comprises a second metal (M2) at a surface of the support, wherein the M1 metal comprises the Pt and the M2 metal comprises a d-block metal, and wherein the M1 metal is not the same metal as the M2 metal.

15. The process as set forth in claim 14, wherein the M2 metal is selected from the group consisting of manganese, iron, and cobalt.

## Patentansprüche

1. Verfahren zur Herstellung von Glucarsäure oder deren Lacton, wobei das Verfahren Folgendes umfasst:
Umsetzen von Glucose mit Sauerstoff in Gegenwart eines heterogenen Oxidationskatalysators, der Pt umfasst, um mindestens einen Teil der Glucose in Glucarsäure oder deren Lacton umzuwandeln,
wobei der pH-Wert der Reaktionsmischung weniger als 7 beträgt,
wobei die Reaktion im Wesentlichen in Abwesenheit einer zugesetzten Base durchgeführt wird,
wobei die Reaktionsmischung bei einer Temperatur von 70°C bis 150°C und unter einem Sauerstoffpartialdruck von mindestens 60 psia (414 kPa) gehalten wird, und
wobei der heterogene Oxidationskatalysator einen Katalysatorträger umfasst, der aus der Gruppe ausgewählt ist, die aus Kohlenstoff, Aluminiumoxid, Siliciumdioxid, Titandioxid, Zirkoniumdioxid und Zeolith besteht.

2. Verfahren nach Anspruch 1, wobei mindestens ein Teil der Glucose in ein Glucarolacton umgewandelt wird.

3. Verfahren nach Anspruch 1 oder 2, wobei der Katalysatorträger Titandioxid umfasst.

4. Verfahren nach einem der Ansprüche 1 bis 3, wobei der Katalysatorträger Zirkoniumdioxid umfasst.

5. Verfahren nach einem der Ansprüche 1 bis 4, bei dem der Katalysatorträger Kohlenstoff umfasst.

6. Verfahren nach einem der Ansprüche 1 bis 5, wobei der Katalysatorträger Ruß umfasst.

7. Verfahren nach einem der Ansprüche 1 bis 6, wobei Pt 0,25 % bis 10 % des Gesamtgewichts des heterogenen Oxidationskatalysators ausmacht.

8. Verfahren nach einem der Ansprüche 1 bis 7, wobei das Reaktionsgemisch bei einer Temperatur von 80°C bis 120°C gehalten wird.

9. Verfahren nach einem der Ansprüche 1 bis 8, wobei der Partialdruck des Sauerstoffs bis zu 1000 psia (6895 kPa) beträgt.

10. Verfahren nach einem der Ansprüche 1 bis 9, wobei die Glucose aus einer Kohlenhydratquelle gewonnen wird.

11. Verfahren nach einem der Ansprüche 1 bis 10, wobei das Reaktionsgemisch im Wesentlichen frei von Stickstoff als aktivem Reaktionsbestandteil ist.

12. Verfahren nach einem der Ansprüche 1 bis 11, wobei der anfängliche pH-Wert der Reaktionsmischung weniger als 6 beträgt.

13. Verfahren nach einem der Ansprüche 1 bis 12, wobei die Reaktion in einem oder mehreren kontinuierlichen Festbettreaktoren durchgeführt wird.

14. Verfahren nach einem der Ansprüche 1 bis 13, wobei der Katalysator ein erstes Metall (Ml) und ferner ein zweites Metall (M2) an einer Oberfläche des Trägers umfasst, wobei das Ml-Metall das Pt umfasst und das M2-Metall ein d-Block-Metall umfasst und wobei das Ml-Metall nicht das gleiche Metall wie das M2-Metall ist.

15. Verfahren nach Anspruch 14, wobei das M2-Metall aus der Gruppe bestehend aus Mangan, Eisen und Kobalt ausgewählt ist.

## Revendications

1. Procédé de préparation d'acide glucarique ou de lactone de celui-ci, le procédé comprenant :
la réaction du glucose avec de l'oxygène en présence d'un catalyseur d'oxydation hétérogène comprenant du Pt pour convertir au moins une partie du glucose en acide glucarique ou en lactone de celui-ci,
dans lequel le pH du mélange réactionnel est inférieur à 7,
dans lequel la réaction est conduite en l'absence substantielle de base ajoutée,
dans lequel le mélange réactionnel est maintenu à une température de 70°C à 150°C et à une pression partielle d'oxygène qui est d'au moins 60 psia (414 kPa), et
dans lequel le catalyseur d'oxydation hétérogène comprend un support de catalyseur choisi dans le groupe constitué du carbone, de l'alumine, de la silice, de l'oxyde de titane, de la zircone et de la zéolite.

2. Procédé selon la revendication 1, dans lequel au moins une partie du glucose est convertie en une glucarolactone.

3. Procédé selon la revendication 1 ou 2, dans lequel le support de catalyseur comprend de l'oxyde de titane.

4. Procédé selon l'une quelconque des revendications 1 à 3, dans lequel le support de catalyseur comprend de la zircone.

5. Procédé selon l'une quelconque des revendications 1 à 4, dans lequel le support de catalyseur comprend du carbone.

6. Procédé selon l'une quelconque des revendications 1 à 5, dans lequel le support de catalyseur comprend du noir de carbone.

7. Procédé selon l'une quelconque des revendications 1 à 6, dans lequel le Pt constitue de 0,25 % à 10 % du poids total du catalyseur d'oxydation hétérogène.

8. Procédé selon l'une quelconque des revendications 1 à 7, dans lequel le mélange réactionnel est maintenu à une température de 80°C à 120°C.

9. Procédé selon l'une quelconque des revendications 1 à 8, dans lequel la pression partielle d'oxygène va jusqu'à 1 000 psia (6 895 kPa).

10. Procédé selon l'une quelconque des revendications 1 à 9, dans lequel le glucose est obtenu à partir d'une source de glucides.

11. Procédé selon l'une quelconque des revendications 1 à 10, dans lequel le mélange réactionnel est sensiblement exempt d'azote en tant que constituant réactionnel actif.

12. Procédé selon l'une quelconque des revendications 1 à 11, dans lequel le pH initial du mélange réactionnel est inférieur à 6.

13. Procédé selon l'une quelconque des revendications 1 à 12, dans lequel la réaction est conduite dans un ou plusieurs réacteurs continus à lit fixe.

14. Procédé selon l'une quelconque des revendications 1 à 13, dans lequel le catalyseur comprend un premier métal (M1) et comprend en outre un second métal (M2) sur une surface du support, dans lequel le métal M1 comprend le Pt et le métal M2 comprend un métal du bloc d, et dans lequel le métal M1 n'est pas le même métal que le métal M2.

15. Procédé selon la revendication 14, dans lequel le métal M2 est choisi dans le groupe constitué du manganèse, du fer et du cobalt.
